# EUROPEAN PATENT SPECIFICATION

(11) **EP 2 229 974 B1**
(45) Date of publication and mention of the grant of the patent: **05.12.2018**
(21) Application number: 09702905.2
(22) Date of filing: 14.01.2009
(51) Int. Cl.: A61M 25/01

(54) **GUIDE WIRE**
FÜHRUNGSDRAHT
FIL DE GUIDAGE

(30) Priority: 18.01.2008 JP 2008009723
(43) Date of publication of application: 22.09.2010
(73) Proprietor: Terumo Kabushiki Kaisha, Tokyo 151-0072 (JP)
(72) Inventor: KINOSHITA, Yasushi, Shizuoka 418-0015 (JP); KOUSAI, Tadashi, Shizuoka 418-0015 (JP); KOBAYASHI, Junichi, Shizuoka 418-0015 (JP)
(74) Representative: Dossmann, Gérard
(86) International application number: PCT/JP2009/050395
(87) International publication number: WO 2009/090963

(56) References cited:
- EP-A1- 1 607 035
- EP-A2- 1 348 461
- WO-A1-2004/084712
- WO-A1-2006/065909
- JP-A- 10 099 442
- JP-A- 2001 046 508
- JP-A- 2004 229 947
- JP-A- 2007 097 662
- JP-A- 2008 264 498
- US-A- 5 084 022
- US-A- 5 379 779

## Description

### [Technical Field]

The present invention relates to a guide wire.

### [Background Art]

At the time of inserting a catheter into an intracorporeal lumen such as a digestive tract and a blood vessel, a guide wire is used to guide the catheter to a target site of the intracorporeal lumen. The guide wire is used in the state of being inserted in the catheter.

In addition, observation or treatment of an intracorporeal lumen or the like by use of an endoscope is also conducted, and a guide wire is again used by which the endoscope or a catheter inserted in the lumen of the endoscope is guided to the target site of the intracorporeal lumen or the like.

In use of the guide wire, an operation of moving the guide wire along the axial direction thereof and an operation of rotating the guide wire about its axis are conducted at the time of inserting the guide wire. These operations are carried out while visually checking the guide wire under fluoroscopy or through an endoscope. If the guide wire is monochromatic, it cannot be seen whether or not the guide wire is displaced (moved) assuredly according to each operation. In view of this, some guide wires are provided at their surfaces with markers (marks) for indicating the position or the like (see, for example, Patent Document 1). The mark given to the guide wire described in Patent Document 1 is helical in shape.

The guide wire described in Patent Document 1, however, has a problem in that, whether the guide wire is moved along its axial direction or rotated about its axis, each portion of the helical mark which is visually checked in practice (each belt-like portion hatched in FIG. 3(b) of Patent Document 1) appears to be moving (changing) in one direction (for example, in the distal direction). Therefore, even when the guide wire is rotated about its axis by applying a torque thereto, the operator would have an illusion that the guide wire is moving distally or proximally, against the operator's will to cause rotation. The illusion may cause a mis-steering of the guide wire. Thus, the guide wire described in Patent Document 1 is poor in steerability.

### [Patent Document 1]

Japanese Patent Laid-open No. 2001-46508
See further EP1348461 A2,JP2007097662 A.

### [Disclosure of the Invention]

It is an object of the present invention to provide a guide wire which is excellent in steerability, particularly, which permits assured confirmation of rotation when the guide wire is rotated about its axis, and which makes it possible to securely prevent the operator from taking the rotating motion as a movement along the axial direction.

The above object can be attained by the present invention which includes the following.

A guide wire, as claimed in claim 1, including: an elongated wire body; and a marker which is provided at at least a distal-side portion of the wire body over the whole circumference and which has a function of indicating the intracorporeal position of the wire body, wherein the marker has a first line-form portion and a second line-form portion which intersect each other at a plurality of locations to have a grid-like shape as a whole.

For this reason, it is excellent in steerability, particularly, it permits assured confirmation of rotation when the guide wire is rotated about its axis, and it makes it possible to securely prevent the operator from taking the rotating motion as a movement along the axial direction.

In addition, in the guide wire according to the present invention, the marker, preferably, has a function of ensuring that when the guide wire is rotated about the axis thereof, the rotation can be confirmed extracorporeally.

For this reason, when the guide wire is rotated about its axis by applying a torque thereto, the fact that "the guide wire is rotated" can be securely confirmed (grasped).

In addition, in the guide wire according to the present invention, the marker, preferably, has a function of ensuring that when the guide wire is moved along the axial direction thereof, the movement can be confirmed extracorporeally.

For this reason, when the guide wire, for example, is pushed in the distal direction and is thereby moved along the axial direction thereof, the fact that "the guide wire has been moved" can be confirmed (grasped) assuredly.

In addition, in the guide wire according to the present invention, the marker, preferably, has a function of ensuring that when the guide wire is moved along the axis thereof or rotated about the axis thereof, whether the displacement is movement or rotation can be distinguished extracorporeally.

For this reason, it is possible to securely distinguish whether the practical displacement of the guide wire is movement of rotation so that the visibility of the marker changes.

In addition, in the guide wire according to the present invention, the first line-form portion, preferably, has a helical shape, and the second line-form portion has a helical shape with a winding direction of helix opposite to the winding direction of helix of the first line-form portion.

For this reason, the first line-form portion and the second line-form portion are provided over the whole circumference of the wire body. As a result, the marker forms grid-like shape as a whole.

In addition, in the guide wire according to the present invention, the pitch of helix of the first line-form portion, preferably, is equal to or different from the pitch of helix of the second line-form portion.

When the pitch of helix of the first line-form portion are the same of the pitch of helix of the second line-form portion, the plurality of the intersecting parts which are intersected the first line-form portion and the second line-form portion are favorably dispersed in the region in which the marker is formed, and, therefore, visibility of the intersecting parts is enhanced. Besides, when the pitch of helix of the first line-form portion are the different of the pitch of helix of the second line-form portion, it is possible to set the number of formations of the intersecting parts where the first line-form portion and the second line-form portion intersect comparatively a lot.

In addition, in the guide wire according to the present invention, the first line-form portion and the second line-form portion, preferably, each have a part where the pitch of helix is gradually decreased along the distal direction.

For this reason, by checking the portion where is gradually decreased along the distal direction, it is possible to grasp that the wire body is thinner (smaller in diameter) and more easily deformable (higher in flexibility) at that portion.

In addition, in the guide wire according to the present invention, the first line-form portion and the second line-form portion, preferably, each have a part where the width thereof is gradually decreased along the distal direction.

For this reason, when the size of the intersecting parts where the first line-form portion and the second line-form portion intersect is set comparatively small, it is effective.

In addition, in the guide wire according to the present invention, the first line-form portion and the second line-form portion, preferably, are the same with or different from each other in mean width.

For this reason, when the mean width of the first line-form portion and the second line-form portion are same with each other, in forming the marker, an operation of forming the line-form portions while setting different widths can be omitted. Therefore, the marker can be formed easily. Besides, when the mean width of the first line-form portion and the second line-form portion are different from each other, it is possible that the size of the intersecting parts where the first line-form portion and the second line-form portion intersect is set comparatively large. Therefore, the visibility of the each intersecting parts is increased.

In addition, in the guide wire according to the present invention, the first line-form portion and the second line-form portion, preferably, each have a mean width of 0.5 to 2 times the mean diameter of the wire body.

For this reason, when the width exceeds the upper limit of the numerical value range just-mentioned, halation may occur, depending on the intensity of light radiated against the marker at the time of visually checking the marker.

In addition, in the guide wire according to the present invention, the first line-form portion and the second line-form portion, preferably, are different from each other in color.

For this reason, in the case where the first line-form portion and the second line-form portion are different from each other in color, when the guide wire is rotated about its axis, the rotating direction can be grasped as follows. If the first line-form portion and the second line-form portion are visually recognized as moving away from each other, the rotation in question is found to be rotation in one direction of about its axis. On the other hand, if the first line-form portion and the second line-form portion are visually recognized as moving closer to each other, the rotation is found to be rotation in the opposite direction, namely, in the direction opposite to said one direction.

In addition, in the guide wire according to the present invention, the marker, preferably, has a structure in which intersecting parts of the first line-form portion and the second line-form portion are greater in height than the other parts.

For this reason, when the guide wire, for example, is inserted into the lumen of an endoscope, the area of contact between the guide wire and the lumen of an endoscope is reduced, and the frictional resistance (sliding resistance) is thereby reduced, leading to enhanced slidability and good steerability of the guide wire.

In addition, in the guide wire according to the present invention, the first line-form portion and the second line-form portion, preferably, are obtained by a method in which one of the line-form portions is formed prior to the other of the line-form portions, and the other of the line-form portions is formed on the one of the line-form portions so that they partly overlap with each other.

For this reason, the intersecting parts where the first line-form portion and the second line-form portion intersect are raised. As a result, it is possible to form easily and surely the marker of the grid-like shapes having the raised intersecting like these.

In addition, in the guide wire according to the present invention, the first line-form portion and the second line-form portion, preferably, are each formed by applying a liquid material to the wire body, followed by drying.

For this reason, it is possible to form easily and surely the marker of the grid-like shapes.

In addition, in the guide wire according to the present invention, at least a part of a portion provided with the marker of the wire body, preferably, has a tapered shape such that the outer diameter thereof is gradually decreased along the distal direction.

This ensures that the marker is disposed on a comparatively highly flexible portion, or an easily deformable portion, of the guide wire (wire body). Consequently, when this portion is curved (deformed), the degree of the curving can be checked assuredly.

In addition, in the guide wire according to the present invention, the marker, preferably, is formed on the outer peripheral side of the wire body, and an intermediate layer is formed between the marker and the wire body.

For this reason, the marker can surely be checked visually.

In addition, in the guide wire according to the present invention, the guide wire, preferably, has a coating layer which coats the marker and the intermediate layer and which has such a transparency that the marker can be visually checked therethrough.

For this reason, the marker can surely be checked visually.

In addition, in the guide wire according to the present invention, the guide wire, preferably, has a coil covering a distal portion of the wire body, and the marker is disposed on the proximal side relative to the coil.

This ensures that positional interference between the coil and the marker can be obviated, and the guide wire is simple in structure.

### [Brief Description of the Drawings]

[FIG. 1] FIG. 1 is a partial longitudinal sectional view showing a first embodiment of the guide wire according to the present invention.
[FIG. 2] FIG. 2 is a longitudinal sectional view of region [A] surrounded by dot-dash line in FIG. 1.
[FIG. 3] FIG. 3 is a perspective view of a marker in the guide wire shown in FIG. 1.
[FIG. 4] FIG. 4 is a sectional view taken along line B-B of FIG. 3.
[FIG. 5] FIG. 5 illustrates the process of change of the marker when the guide wire is rotated about its axis.
[FIG. 6] FIG. 6 illustrates the process of change of the marker when the guide wire is moved along its axial direction.
[FIG. 7] FIG. 7 is a side view showing a second embodiment of the guide wire according to the present invention.
[FIG. 8] FIG. 8 is a side view showing a third embodiment of the guide wire according to the present invention.

Now, the guide wire according to the present invention will be described in detail below, based on preferred embodiments shown in the accompanying drawings.

### <First Embodiment>

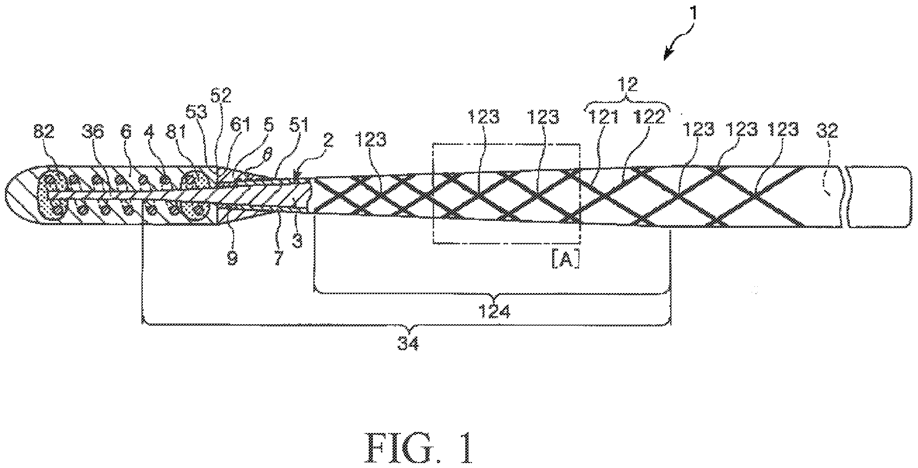

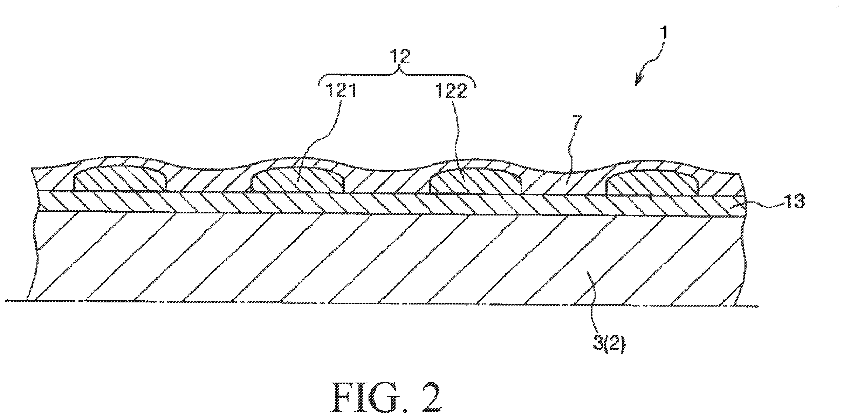

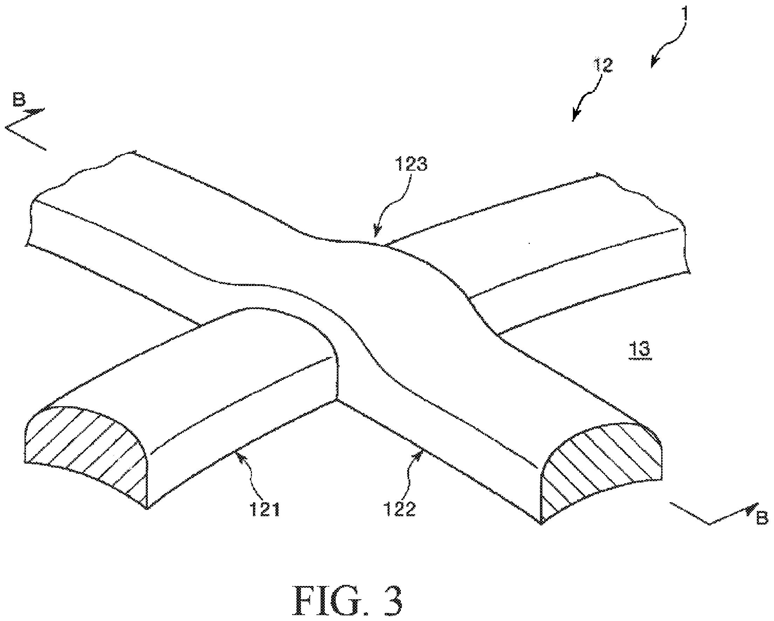

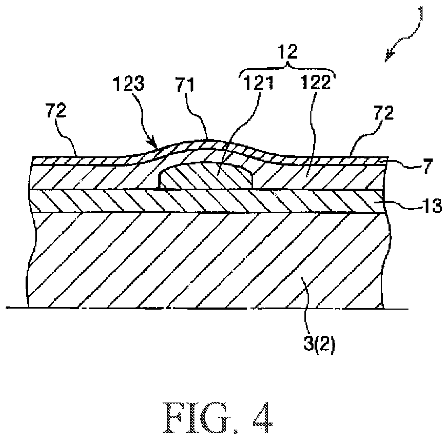

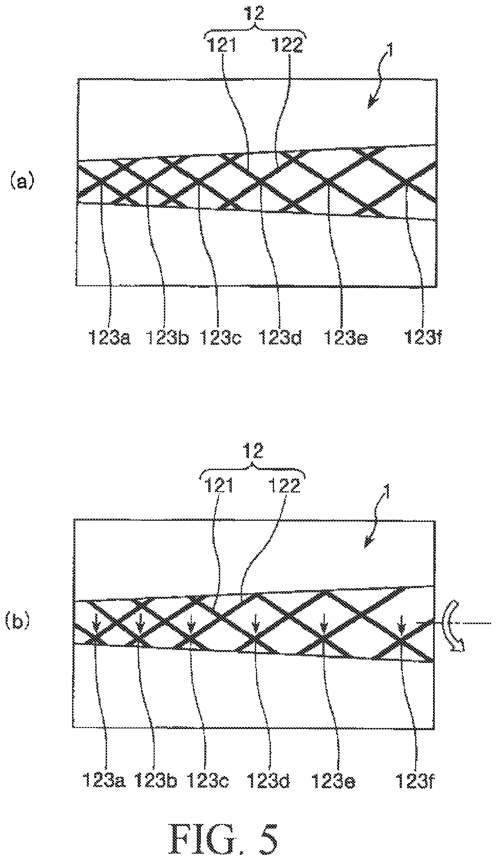

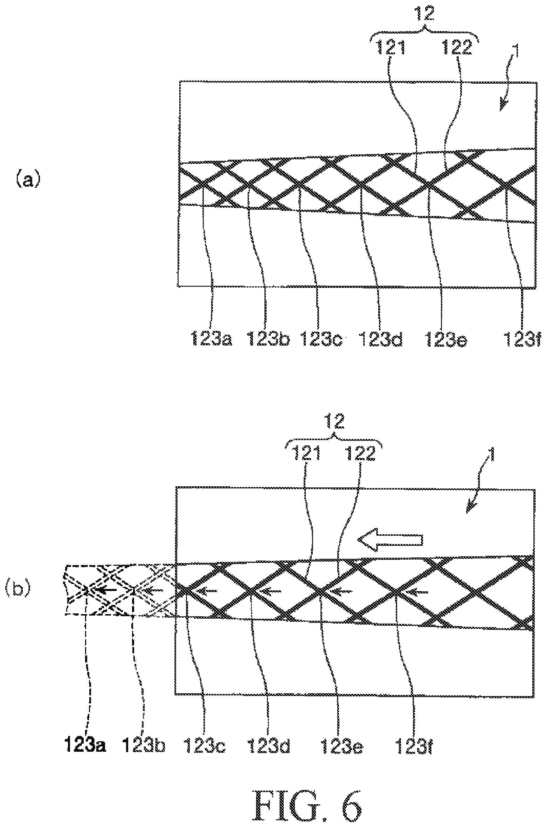

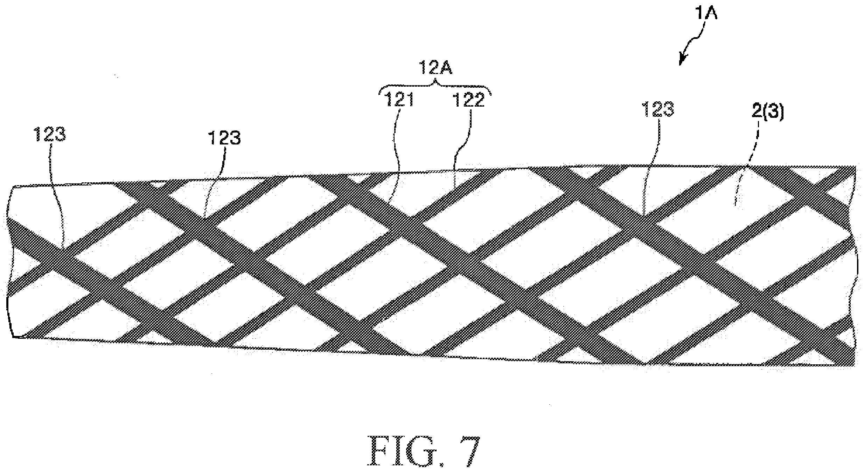

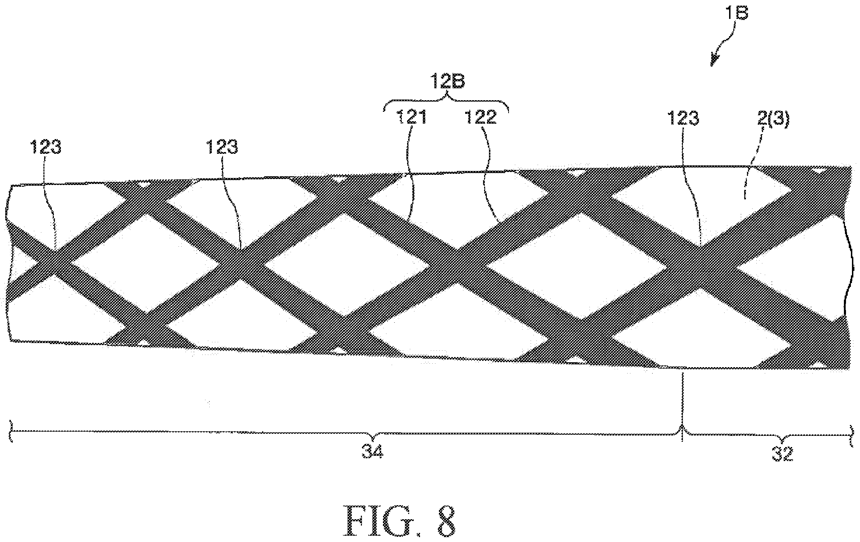

FIG. 1 is a partial longitudinal sectional view showing a first embodiment of the guide wire according to the present invention; FIG. 2 is a longitudinal sectional view of region [A] surrounded by dot-dash line in FIG. 1; FIG. 3 is a perspective view of a marker in the guide wire shown in FIG. 1; FIG. 4 is a sectional view taken along line B-B of FIG. 3; FIG. 5 illustrates the process of change of the marker when the guide wire is rotated about its axis; and FIG. 6 illustrates the process of change of the marker when the guide wire is moved along its axial direction. Incidentally, in the following description, for convenience of description, the right side in FIGS. 1, 2, 5 and 6 (and FIGS. 7 and 8, also) will be referred to as "proximal," and the left side as "distal." Besides, for easier understanding, the guide wire in FIG. 1 is schematically shown in the state of being shortened in the longitudinal direction and being exaggerated in the radial direction, so that the ratio between the longitudinal size and the radial size in the figure is different from the ratio in practice.

The guide wire 1 shown in FIG. 1 is a catheter guide wire to be used in the state of being inserted in the lumen of a catheter (inclusive of an endoscope). The guide wire 1 has a wire body 2 composed of a flexible or pliable core wire (wire) 3, a helical coil 4, a resin coating layer 6, an annular member (step-filling member) 5, and a marker 12.

In this embodiment, the wire body 2 has a single, long, continuous core wire 3. The core wire 3 is circular in cross-sectional shape. It should be noted here, however, the core wire 3 in the present invention is not limited to this one. For example, the core wire 3 may be formed by joining (connecting) a plurality of core wires (wires) of the same or different materials by, for example, welding or soldering. Incidentally, where the core wire 3 is formed by joining two core wires, for example, the joint may be located in any of a body portion 32, a tapered portion 34, and a small-diameter portion 36 which are described later.

The overall length of the guide wire 1 is not particularly limited, and is preferably about 200 to 5000 mm. In addition, the outer diameter (mean value) of the guide wire 1 is not particularly limited, and is preferably about 0.2 to 1.2 mm.

The core wire 3 extends over substantially the entire length of the guide wire 1. The core wire 3 has the body portion 32 corresponding to the body portion of the guide wire 1, the tapered portion (gradually decreased outer diameter portion) 34 located on the distal side of the body portion 32, and the small-diameter portion 36 located on the distal side of the tapered portion 34. The body portion 32 is substantially constant in outer diameter, the tapered portion 34 has an outer diameter gradually decreased along the distal direction (is tapered), and the small-diameter portion 36 is substantially constant in outer diameter.

Since the core wire 3 is provided with the tapered portion 34, the flexibility of the core wire 3 is gradually (continuously) increased along the distal direction from the vicinity of the boundary part between the body portion 32 and the tapered portion 34. As a result, the flexibility of the guide wire 1 is increased, so that steerability and safety at the time of inserting the guide wire 1 into a living body are enhanced.

The marker 12 is formed on the tapered portion 34 from an intermediate part toward the proximal side (see FIG. 1). This ensures that the marker 12 is disposed on a comparatively highly flexible portion, or an easily deformable portion, of the guide wire 1 (wire body 2). Consequently, when this portion is curved (deformed), the degree of the curving can be checked assuredly.

In addition, since the small-diameter portion 36 is provided on the distal side of the tapered portion 34, the distalmost flexible portion can be set long, resulting in that the distalmost portion is more flexible.

Besides, at least a part of the small-diameter portion 36 of the core wire 3 may be a reshapable portion which can be reshaped. The reshapable portion, preferably, is in the shape of a flat plate, a prism or the like.

The outer diameter of the body portion 32 of the core wire 3 is not particularly limited, and is preferably about 0.3 to 1.0 mm, more preferably about 0.4 to 0.7 mm.

The outer diameter of the small-diameter portion 36 of the core wire 3 is not particularly limited, and is preferably about 0.05 to 0.3 mm, more preferably about 0.1 to 0.2 mm. Incidentally, the outer diameter of the small-diameter portion 36 may not necessarily be constant, and the outer diameter may be gradually decreased along the distal direction.

In addition, the length of the tapered portion 34 varies depending on the use and the kind of the guide wire 1, and is not particularly limited. The length of the tapered portion 34 may be preferably about 10 to 300 mm, more preferably about 30 to 250 mm.

Besides, the length of the small-diameter portion 36 is not particularly limited, and may be preferably about 0 to 100 mm, more preferably about 10 to 50 mm.

Incidentally, the taper angle (rate of reduction in outer diameter) of the tapered portion 34 may be constant along the longitudinal direction of the core wire 3 (wire body 2), or may vary at some portion along the longitudinal direction. In addition, the tapered portion 34 is not limited to only one tapered portion; two or more tapered portions may be provided.

Examples of the material which can be used to form the core wire 3 include various metallic materials such as stainless steel, Ni-Ti alloys, Ni-Al alloys, Cu-Zn alloys and other superelastic alloys, and resin materials having comparatively high stiffness, which may be used either singly or combination of two or more of them.

In addition, the coil 4 is disposed around the outer periphery of a distal portion of the core wire 3 (wire body 2), or, in the configuration shown in the figure, around the outer periphery of the small-diameter portion 36 of the core wire 3 and the outer periphery of the portion ranging to an intermediate part of the tapered portion 34. The coil 4 is a member obtained by winding (forming) a wire (thin wire) in a helical shape, and is so disposed as to cover a distal-side portion (outer periphery) of the core wire 3 (wire body 2). In the configuration shown in the figure, the distal-side portion (distal portion) of the core wire 3 is inserted in a substantially central part of the inside of the coil 4. The distal-side portion of the core wire 3 is inserted so as not to make contact with the inner surface of the coil 4.

The proximal end of the coil 4 is located at an intermediate part of the tapered portion 34 of the core wire 3, and the marker 12 is located on the proximal side relative to the intermediate part. This ensures that positional interference between the coil 4 and the marker 12 can be obviated, and the guide wire 1 is simple in structure. Incidentally, the marker 12 may be formed ranging to the outer peripheral side of the coil 4. In other words, the marker 12 may be so formed as to overlap with the coil 4 in side view.

Incidentally, in the configuration shown in the figure, the coil 4 is so set that gaps are left between adjacent windings of the wire wound in a helical form, in the condition where no external force is applied thereto. Unlike in the configuration shown, however, the coil 4 may be so set that the windings of the wire wound in a helical form are disposed closely, without any gap therebetween, in the condition where no external force is applied thereto.

The coil 4, preferably, is formed of a metallic material. Examples of the metallic material for use to form the coil 4 includes stainless steel, superelastic alloys, cobalt alloys, noble metal such as gold, platinum, tungsten, etc., alloys containing these noble metals (for example, platinum-iridium alloys), and so on. Particularly, where the coil 4 is formed of a radiopaque material (a material which has radiopacity) such as noble metals, the guide wire 1 can have radiopacity, so that the guide wire 1 can be inserted into a living body while checking the position of its distal portion under fluoroscopic observation, which naturally is preferable. In addition, the coil 4 may be formed of different materials on the distal side and on the proximal side. For instance, the coil 4 may be composed of a coil of a radiopaque material on the distal side and a coil of a comparatively radiolucent material (stainless steel or the like) on the proximal side. Incidentally, the overall length of the coil 4 is not particularly limited, and is preferably about 5 to 500 mm. Besides, while the coil 4 is formed by use of a wire circular in cross-section in this embodiment, this configuration is not limitative: the cross-sectional shape of the wire may be other shape, such as an ellipse, a tetragon (especially, a rectangle), etc.

A proximal portion and a distal portion of the coil 4 are fixed (firmly attached) to the core wire 3 by fixing materials 81 and 82, respectively.

These fixing materials 81 and 82, i.e., the two fixing portions for fixing the core wire 3 and the coil 4 to each other, are provided on the distal side relative to the annular member 5 (described later), and are not in contact with the annular member 5. This makes it possible to prevent conduction between the core wire 3 and the annular member 5 from being made through the fixing material 81. Accordingly, conduction between the outer surface of the guide wire and the core wire 3 can be prevented from occurring.

The fixing materials 81 and 82 are each composed of a solder (brazing metal). Incidentally, the material constituting each of the fixing materials 81 and 82 is not limited to a solder, and may be an adhesive, for example. Further, the method of fixing the coil 4 is not limited to the use of the fixing materials; for example, welding may be adopted as the fixing method.

In addition, the guide wire 1 has the resin coating layer 6 which covers a distal portion of the core wire 3 (wire body 2), the coil 4, and the outer peripheries (outer surfaces) of the fixing materials 81 and 82. The resin coating layer 6 is in intimate contact with the outer periphery of the distal portion of the core wire 3.

Incidentally, while the resin coating layer 6 have entered into the inside of the coil 4 in the configuration shown in the figure, the resin coating layer 6 may not necessarily have entered into the inside of the coil 4.

The resin coating layer 6 can be formed for various purposes. An example of the purpose is to enhance safety in inserting the guide wire 1 into a blood vessel or the like. For this purpose, the resin coating layer 6 is preferably composed of a material rich in flexibility (a soft material, an elastic material). Examples of the material include polyolefins such as polyethylene, polypropylene, etc., polyvinyl chloride, polyesters (PET, PBT, etc.), polyamides, polyimides, polyurethane, polystyrene, silicone resins, thermoplastic elastomers such as polyurethane elastomer, polyester elastomer, polyamide elastomer, etc., various rubber materials such as latex rubber, silicone rubber, etc., and composite materials obtained by combining two or more of these materials.

Especially, where the resin coating layer 6 is composed of one of the above-mentioned thermoplastic elastomers and various rubbers, the flexibility of the distal portion of the guide wire 1 is more enhanced. In this case, therefore, it is possible to more securely prevent the distal portion from injuring the inner wall of a blood vessel or the like at the time of insertion into the blood vessel or the like and ensures very high safety.

Besides, particles (filler) composed of a radiopaque material (a material which have radiopacity) may be dispersed in the resin coating layer 6. This ensures that the guide wire 1 can have radiopacity, so that the guide wire 1 can be inserted into a living body while checking the position of its distal portion under fluoroscopic observation. The material constituting the particles is not particularly limited, provided it is a radiopaque material. For example, noble metals such as gold, platinum, tungsten, etc. and alloys containing them (for example, platinum-iridium alloys) can be used as the material of the particles.

The thickness of the resin coating layer 6 is not particularly limited, and may be appropriately set in consideration of the purpose of forming the resin coating layer 6, the material(s) constituting the layer, the method of forming the layer, and so on. Usually, the thickness (mean value) is preferably about 30 to 300 µm, more preferably about 50 to 200 µm. When the thickness of the resin coating layer 6 is too small, the purpose of forming the resin coating layer 6 may be attained insufficiently. On the other hand, when the thickness of the resin coating layer 6 is too large, it may influence the physical properties of the wire body 2 (guide wire 1). Incidentally, the resin coating layer 6 may be a laminate of two or more layers.

In addition, the distal surface of the resin coating layer 6 is rounded. This makes it possible to more securely prevent the distal surface of the resin coating layer 6 (guide wire 1) from injuring the inner surface of a blood vessel or the like at the time of inserting the guide wire 1 into the blood vessel or the like.

The guide wire 1 is provided, on the proximal side of the resin coating layer 6, with the annular member 5 so as to fill up the stepped space between the proximal portion of the resin coating layer 6 and the wire body 2. Incidentally, the outer diameter of the proximal end of the resin coating layer 6 is greater than the outer diameter of the wire body 2 at the proximal end of the resin coating layer 6, and the stepped space is generated due to the difference in outer diameter.

Besides, the outer diameter of the distal end 52 of the annular member 5 is substantially equal to the outer diameter of the proximal end of the resin coating layer 6, and the distal end face 53 of the annular member 5 is jointed (adhered) to the proximal end face 61 of the resin coating layer 6. In this case, the resin coating layer 6 is prevented from extending toward the proximal side beyond the distal end 52 of the annular member 5 to coat the annular member 5. In other words, a continuous surface without any step is formed between the distal end 52 of the annular member 5 and the proximal end of the resin coating layer 6.

In addition, the outer diameter of the annular member 5 is gradually decreased from the distal side toward the proximal side (along the proximal direction), and the outer diameter of the annular member 5 is smaller at the proximal end 51 than at the distal end 52. The outer diameter of the proximal end 51 of the annular member 5 is substantially equal to the outer diameter of the wire body 2 (coating layer 7) at the proximal end 51 of the annular member 5. In other words, a continuous surface free of any step is formed between the wire body 2 (coating layer 7) and the proximal end 51 of the annular member 5. The outer diameter of the proximal end 51 of the annular member 5 is smaller than the outer diameter of the body portion 32 of the core wire 3. The annular member 5 has a length of 0.5 to 15 mm.

Besides, the inner diameter of the annular member 5 is larger at the proximal end 51 than at the distal end 52. This is because the annular member 5 is located on the tapered portion 34 of the core wire 3, as will be described later. Incidentally, the inner diameter at the proximal end 51 may be equal to the inner diameter at the distal end 52.

Owing to the presence of the annular member 5, the proximal portion of the resin coating layer 6 can be prevented from being caught by a medical implement used in combination with the guide wire 1, for example, by the distal end of a catheter or by a riser base of an endoscope, and, therefore, the resin coating layer 6 can be prevented from being peeled off. In addition, the slidability of the guide wire 1 can be prevented from being lowered due to the above-mentioned step.

Besides, the inclination angle θ (taper angle) (rate of reduction in outer diameter) of the annular member 5, in this embodiment, is constant along the longitudinal direction of the core wire 3 (wire body 2). Incidentally, the inclination angle θ may vary along the longitudinal direction at some portions. The inclination angle θ is preferably not more than 30°, more preferably about 2 to 25°, and further preferably about 5 to 20°. This ensures that the annular member 5 can be prevented from being caught by a medical implement used in combination with the guide wire 1, for example, by the distal end of a catheter or by a riser base of an endoscope.

In addition, the hardness of the annular member 5 is preferably set to be higher than the hardness of the resin coating layer 6. This also ensures that the annular member 5 can be prevented from being caught by a medical implement used in combination with the guide wire 1, for example, by the distal end of a catheter or by a riser base of an endoscope.

Besides, one or both of the distal end face 53 and the inner peripheral surface of the annular member 5 may be roughened. When the distal end face 53 of the annular member 5 is roughened, its adhesion to the resin coating layer 6 is enhanced. When the inner peripheral surface is roughened, its adhesion to the fixing member 9 (described later) is enhanced.

In addition, the material constituting the annular member 5 is not particularly limited; for example, various resin materials and various metallic materials and the like can be used as the material. For instance, the same material as that of the resin coating layer 6 may be used, or a material different from the material of the resin coating layer 6 may be used.

It should be noted here that the annular member 5 is preferably formed by use of a metallic material (metal) or a resin material (resin), particularly a metallic material. Examples of the metallic material which can be used to form the annular member 5 include stainless steel, titanium, titanium alloys, Ni-Ti alloys, aluminum, gold, and platinum. When a noble metal such as gold and platinum or an alloy thereof is used, radiopacity is enhanced. In addition, where the annular member 5 is formed by use of a metallic material, the outer periphery of the annular member 5 may be covered with a coating layer (not shown). The material constituting the coating layer is not particularly limited; for example, various resin materials, various ceramics, various metallic materials and the like can be used; particularly, an insulating material is used preferably. Besides, where the annular member 5 is formed by use of a hard resin material, examples of the hard resin material include polycarbonate, polyamide (nylon), polyethylene terephthalate, polyacetal, and polyphenylene sulfide.

In addition, the annular member 5 is fixed (firmly attached) to the core wire 3 (wire body 2) by the fixing material 9 provided on the outer periphery of the core wire 3 (wire body 2).

The fixing material 9, preferably, is composed of an adhesive, particularly an insulating adhesive. This makes it possible to insulate the core wire 3 and the annular member 5 from each other. Consequently, for example in the case where a medical implement used by passing an electric current is disposed along the guide wire 1, troubles such as leakage of current from the outer surface of the annular member 5 can be prevented from occurring.

Incidentally, the fixing material 9 is not limited to an adhesive. For example, in the case where the annular member 5 is formed by use of a metallic material, a solder (brazing metal) or the like may be used as the fixing material 9. In addition, the method of fixing the annular member 5 is naturally not limited to the use of the fixing material.

Besides, the annular member 5 is located at the tapered portion 34 of the core wire 3 (wire body 2). While the annular member 5 is entirely located at the tapered portion 34 in the configuration shown in the figure, this is not limitative. A configuration may also be adopted in which only a part of the annular member 5 is located at the tapered portion 34.

The annular member 5 functions to moderate the difference in rigidity (flexural rigidity, torsional rigidity) of the wire body 2 between the proximal side and the distal side of the annular member 5. Specifically, as above-mentioned, the outer diameter of the core wire 3 at the tapered portion 34 decreases gradually along the distal direction, and the rigidity thereof is gradually lowered along the distal direction. On the other hand, the wire body 2 is provided with the resin coating layer 6 on the distal side of the annular member 5. Where the annular member 5 is not provided, the rigidity increases abruptly at the proximal end of the resin coating layer 6, so that kinking is liable to occur. In this guide wire 1, however, the annular member 5 is provided to ensure that the abrupt increase in rigidity at the proximal end of the resin coating layer 6 is prevented, whereby kinking at the proximal end of the resin coating layer 6 can be prevented from occurring.

In the present invention, the guide wire 1 is applicable not only to guide wires used under fluoroscopy but also to guide wires used together with an endoscope. More specifically, the guide wire 1 of the invention is applicable to a guide wire by which a catheter inserted in the lumen of an endoscope is guided to a target site of an intracorporeal lumen or the like (hereinafter, such a guide wire will be referred to as "trans-endoscopic guide wire"). In this embodiment, the case where the guide wire 1 is applied to a trans-endoscopic guide wire will be described representatively.

In the trans-endoscopic guide wire, the guide wire 1 is provided at the outer surface thereof with a visual marker which functions to indicate the intracorporeal position of the guide wire 1 (wire body 2), and the visual marker is visually recognized through an endoscope. In this embodiment, the marker 12 corresponds to the visual marker. The marker 12 is provided at the outer periphery of the wire body 2 through the primary layer (intermediate layer) 13 therebetween (see FIG. 2). Besides, the guide wire 1 is provided also with the coating layer 7 covering the marker 12 and the primary layer (see FIG. 2).

As shown in FIG. 1, the marker 12 includes a first line-form portion 121 and a second line-form portion 122.

The first line-form portion 121 is in the form of being wound helically. This results in that the first line-form portion 121 is provided over the whole circumference of the wire body 2. In addition, the first line-form portion 121 is in the form of loose winding in which gaps are left between the adjacent lines.

The second line-form portion 122 is also in a helical shape, like the first line-form portion 121, but the winding direction of helix thereof is opposite to the winding direction of helix of the first line-form portion 121. As a result, the second line-form portion 122 is provided over the whole circumference of the wire body 2. Besides, like the first line-form portion 121, the second line-form portion 122 is in the form of loose winding in which gaps are left between the adjacent lines.

With the first line-form portion 121 and the second line-form portion 122 provided in this manner, the line-form portions intersect each other at a plurality of locations, so that the marker 12 as a whole is grid-like in shape.

Incidentally, as shown in FIG. 2, the first line-form portion 121 and the second line-form portion 122 are each upheaved from the primary layer 13 and each have a semi-elliptic shape and a convexly curved top portion in vertical sectional view. Besides, the height of the first line-form portion 121 and the second line-form portion 122 is not particularly limited, and is preferably, for example, 3 to 8 µm, more preferably 3 to 5 µm.

When the guide wire 1 is extracorporeally observed through an endoscope, the marker 12 appears as shown in FIGS. 5 and 6. A case where the guide wire 1 is rotated about its axis will be described referring to FIG. 5, and a case where the guide wire 1 is moved in its axial direction will be described referring to FIG. 6.

First, the case where the guide wire 1 is rotated about its axis will be described.

FIG. 5(a) shows the condition before rotation of the guide wire 1. When the guide wire 1 then rotated by a predetermined amount (angle) in the direction of arrow in the figure, the condition shown in FIG. 5(b) results.

As above-mentioned, the marker 12 has a plurality of intersecting parts (intersections) 123 where the first line-form portion 121 and the second line-form portion 122 intersect each other. Here, paying attention to intersecting parts 123a, 123b, 123c, 123d, 123e and 123f of the first line-form portion 121 and the second line-form portion 122 which can be observed (as in FIG. 5(a)) in practice, the intersecting parts 123a to 123f in FIG. 5(b) have been moved downwards in the figure, as compared with those in the condition shown in FIG. 5(a).

The visual checking of the marker 12 in this manner ensures that when the guide wire 1 is rotated about its axis by applying a torque to the guide wire 1, the fact that "the guide wire 1 has been rotated" can be securely confirmed (grasped).

In addition, when the guide wire 1 is rotated in the direction opposite to the above, the intersecting parts 123a to 123f are moved upwards in the figure from the condition shown in FIG. 5(a). This makes it possible to confirm whether the guide wire 1 is being rotated upwards or downwards in the figure, i.e., to confirm the rotating direction of the guide wire 1.

Now, the case where the guide wire 1 is moved in the axial direction thereof will be described.

FIG. 6(a) shows the condition before movement of the guide wire 1. When the guide wire 1 is moved by a predetermined amount (distance) in the direction of arrow in the figure, the condition shown in FIG. 6(b) results.

Here, paying attention to the intersecting parts 123a to 123f of the first line-form portion 121 and the second line-form portion 122 which can be observed (as in FIG. 6(a)) in practice, the intersecting parts 123a to 123f in FIG. 6(b) have been moved in the distal direction (leftwards in the figure) from the condition shown in FIG. 6(a). In addition, the intersecting parts 123a and 123b come out of (disappear from) the visual angle (field of view).

Since the marker 12 can thus be visually checked, it is ensured that when the guide wire 1 is pushed in the distal direction and is thereby moved along the axial direction thereof, the fact that "the guide wire 1 has been moved" can be confirmed (grasped) assuredly.

In addition, when the guide wire 1 is moved in the direction opposite to the above by pulling it, the intersecting parts 123a to 123f are moved in the proximal direction (rightwards in the figure) from the condition shown in FIG. 6(a). As a result, it is possible to confirm whether the guide wire 1 is being moved in the distal direction or the proximal direction, i.e., to confirm the moving direction of the guide wire 1.

As has been described above, when the guide wire 1 is moved along its axis or rotated about its axis, the above-mentioned change of the marker 12 makes it possible to securely distinguish whether the practical displacement of the guide wire 1 is a movement or rotation.

Besides, in the case of a guide wire having a single helical marker as in the related art, even if the guide wire is rotated about its axis by applying a torque thereto, the operator would have an illusion that the guide wire is being advanced or retracted (is being moved), against the operator's will to cause rotation. In using the guide wire 1, on the other hand, such an illusion (trouble) can be securely prevented from occurring, and excellent steerability is ensured.

As shown in FIG. 1, at the same position in the axial direction of the wire body 2, specifically, the body portion 32 and the tapered portion 34 of the wire body 2, the pitch of helix of the first line-form portion 121 and the pitch of helix of the second line-form portion 122 are equal. This results in that the plurality of intersecting parts 123 are favorably dispersed in the region in which the marker 12 is formed, and, therefore, visibility of the intersecting parts 123 is enhanced. Besides, there is a merit in that the rotation of the guide wire 1 and the pushing/pulling operation (movement) of the guide wire 1 can be easily discriminated from each other.

In addition, the marker 12 is provided with a decreasing-pitch portion 124 where the pitch of helix of the first line-form portion 121 and the second line-form portion 122 in the tapered portion 34 is gradually decreased along the distal direction. By checking the decreasing-pitch portion 124, it is possible to grasp that the wire body 2 is thinner (smaller in diameter) and more easily deformable (higher in flexibility) at that portion.

As shown in FIG. 1 (and in FIGS. 5 and 6, also), the width (mean width) of the first line-form portion 121 and the width (mean width) of the second line-form portion 122 are equal. This ensures that, in forming the marker 12, an operation of forming the line-form portions while setting different widths can be omitted. Therefore, the marker 12 can be formed easily.

Incidentally, the width of the first line-form portion 121 and the second line-form portion 122 is preferably 0.5 to 2 times, more preferably 0.5 to 1.5 times, the mean diameter of the wire body 2. When the width exceeds the upper limit of the numerical value range just-mentioned, halation may occur, depending on the intensity of light radiated from the endoscope at the time of visually checking the marker 12.

Besides, the first line-form portion 121 and the second line-form portion 122 may be the same or different in color. Preferably, however, the line-form portions are different in color. In the case where the first line-form portion 121 and the second line-form portion 122 are different from each other in color, when the guide wire 1 is rotated about its axis, the rotating direction can be grasped as follows. If the first line-form portion 121 and the second line-form portion 122 are visually recognized as moving away from each other, the rotation in question is found to be rotation in the direction of arrow in FIG. 5. On the other hand, if the first line-form portion 121 and the second line-form portion 122 are visually recognized as moving closer to each other, the rotation is found to be rotation in the opposite direction, namely, in the direction opposite to the direction of arrow in FIG. 5.

As shown in FIG. 3, in the marker 12, the height of the intersecting parts 123 is greater than the height of the other portions, i.e., than the height of the portions exclusive of the intersecting parts 123 of the first line-form portion 121 and the second line-form portion 122. Incidentally, the height of the intersecting parts 123 is not particularly limited, and is preferably, for example, 3 to 10 µm, more preferably 5 to 8 µm.

As above-mentioned, the marker 12 is covered with the coating layer 7. The coating layer 7 has an outer surface (the outer surface of the guide wire 1) which is raised in the areas where the intersecting parts 123 (marker 12) are arranged than in the areas where the marker 12 is not arranged. Specifically, raised portions (projections) 71 are formed in the areas where the intersecting parts 123 are arranged, whereas hollowed portions (recesses) 72 are formed in the areas where the marker 12 is not arranged. This is because the thickness of the coating layer 7 is comparatively small, so that the outer surface of the resin layer 7 is influenced by the intersecting parts 123, to be raised correspondingly to (to have a shape corresponding to) the shape and pattern of the intersecting parts 123.

As a result, the area of contact between the outer surface of the coating layer 7 and the lumen of a catheter or the lumen of an endoscope is reduced, and the frictional resistance (sliding resistance) is thereby reduced, leading to enhanced slidability and good steerability of the guide wire 1.

In addition, the raised portions 71 and the hollowed portions 72 are not formed by direct working of the coating layer 7 but are formed under the influence of the intersecting parts 123 underlying the coating layer 7. Therefore, the outer surface of the coating layer 7 is a smooth surface which is free of sharp corner portions or crest portions. Specifically, according to the roundness of the top portions of the first line-form portion 121 and the second line-form portion 122, the raised portions 71 are rounded similarly. This further enhances slidability, and ensures very high safety.

The first line-form portion 121 and the second line-form portion 122 are each formed of a material containing a resin and a pigment. The material constituting the first line-form portion 121 and the material constituting the second line-form portion 122 are substantially the same. Therefore, the material constituting the first line-form portion 121 will be described below representatively.

The color of the first line-form portion 121 is determined mainly by the kind and properties of the pigment contained in the first line-form portion 121, the composition and properties (particularly, tone and the like) of the resin material, and the content of the pigment. Therefore, by regulating these factors, it is possible to freely set the color of the first line-form portion 121.

Here, for recognizing the motion of the guide wire 1 through an endoscope, the color of the first line-form portion 121 is an important factor, and its combination with the color of the primary layer 13 serving as a substrate should be taken into account.

In an exemplary case where the primary layer 13 is silver (metallic color), grey or black in color and the first line-form portion 121 is red or yellow, the difference in brightness of color between them is large (high contrast), leading favorably to a high visibility of the first line-form portion 121. Besides, it is preferable that both of colors have a relation to complement each other, leading favorably to a high visibility of the first line-form portion 121. Besides, it is particularly preferable to select a combination of colors which gives a clear contrast, such as a combination of black or deep color (charcoal grey, dark brown, dark blue, purple, etc.) with yellow, yellowish green, orange or the like and a combination of blue with red, orange, pink or the like. In addition, a combination of akin colors with different shades may also be used, for example, a combination of dark blue with light blue or a combination of reddish brown with pink.

The resin contained in the material constituting the first line-form portion 121 is not particularly limited, and is preferably the following (1) or (2).
(1) The resin contained in the material constituting the first line-form portion 121 may preferably be a resin (heat-resistant resin) having a melting point of not less than 200°C, more preferably a resin having a melting point of about 200 to 300°C.
   Examples of the resin having a melting point of not less than 200°C include polysulfone, polyimide, polyetherether ketone, polyarylene ketone, polyphenylene sulfide, polyarylene sulfide, polyamide-imide, polyetherimide, polyimide-sulfone, polyarylsulfone, polyarylethersulfone, polyester, polyether-sulfone, and fluororesins such as polytetrafluoroethylene (PTFE), ethylenetetrafluoroethylene copolymer (ETFE), etc., which may be used either singly or in combination of two or more of them.
(2) The resin contained in the material constituting the first line-form portion 121 may preferably be a thermosetting resin.

Examples of the thermosetting resin include epoxy resins, phenolic resins, polyesters (unsaturated polyesters), polyimides, silicone resins, polyurethane, etc., which may be used either singly or in combination of two or more of them.

Besides, the content of the pigment in the first line-form portion 121 varies depending on the kind and properties of the pigment and the composition and properties of the resin material. In order to obtain a good color, the content of the pigment is preferably about 10 to 99 wt.%, more preferably about 20 to 50 wt.%, based on the whole weight of the first line-form portion 121.

The pigment in the first line-form portion 121, preferably, is dispersed uniformly. However, the pigment may be locally present, for example, on the outer surface side in the first line-form portion 121. The pigment may be either an inorganic pigment or an organic pigment, the inorganic pigment being preferred from the viewpoint of heat resistance. Examples of the inorganic pigment which can be used include carbon black, mica, titanium dioxide, nickel-titanium yellow, Prussian blue, Milori blue, cobalt blue, ultramarine, viridian, etc. Incidentally, these pigments may be used either singly or in combination (particularly, mixture) of two or more of them. Besides, the mean particle diameter of the pigment is not particularly limited, and is preferably, for example, 0.3 to 5 µm, more preferably 0.5 to 3 µm.

Such a marker 12 can be formed, for example, as follows.

Of the first line-form portion 121 and the second line-form portion 122, the first line-form portion 121 is formed prior to the second line-form portion 122.

In order to form the first line-form portion 121, first, a masking tape is helically wound around and adhered to the portion, other than the region where to form the first line-form portion 121, of the core wire 3 which is to be the wire body 2.

Next, the resin material in a liquid state containing the pigment added thereto (this material will hereinafter be referred to as "liquid material") is supplied to coat (is applied to) the exposed portion, where the masking tape is not wound, of the core wire 3. Examples of the method of coating (applying) include a spraying method and a dipping method.

Subsequently, the liquid material thus applied is dried. Thereafter, the masking tape is peeled off (removed).

By the steps as above, the first line-form portion 121 can be formed. Next, the second line-form portion 122 is formed.

In order to form the second line-form portion 122, first, a masking tape is helically wound around to an opposite direction and adhered to the portion, other than the region where to form the second line-form portion 122, of the core wire 3 which has been provided thereon with the first line-form portion 121, in the same manner as above.

Next, in the same manner as in forming the first line-form portion 121, a liquid material is supplied to coat (is applied to) the exposed portion, where the masking tape is not wound, of the core wire 3.

Subsequently, the liquid material thus applied is dried. Thereafter, the masking tape is peeled off (removed).

By the steps as above, the second line-form portion 122 partly superposed on the first line-form portion 121 can be formed. Therefore, a grid-like marker 12 with the intersecting parts 123 raised can be formed easily and assuredly.

Incidentally, the first line-form portion 121 and the second line-form portions 122 may each be formed either singly or in plurality. In addition, the number of the first line-form portion(s) 121 and the number of the second line-form portion(s) 122 may be the same or different.

Besides, while the first line-form portion 121 and the second line-form portion 122 are the same in width in the configuration shown in FIG. 1, this is not limitative. Namely, the first line-form portion 121 and the second line-form portion 122 may be different in width.

As shown in FIGS. 2 and 4, the coating layer 7 covering the marker 12 and the primary layer 13 is formed. The coating layer 7 has such a degree of transparency that the marker 12 can be visually recognized therethrough.

The coating layer 7 can be formed for any of various purposes. An exemplary purpose is to reduce the friction (sliding resistance) on the guide wire 1 and to enhance slidability of the guide wire 1, thereby enhancing steerability of the guide wire 1.

In order to contrive a reduction in the friction (sliding resistance) on the guide wire 1, the coating layer 7 is preferably formed by use of a material capable of reducing friction as described below. This results in that the frictional resistance (sliding resistance) on the inner wall of a catheter used together with the guide wire 1 is reduced, whereby slidability of the guide wire 1 is enhanced, and steerability of the guide wire 1 in the catheter or in an endoscope is more enhanced. In addition, the lowered sliding resistance of the guide wire 1 ensures that when the guide wire 1 is moved and/or rotated in an endoscope (or in a catheter, similarly), the guide wire 1 can be securely prevented from kinking or twisting.

Besides, the coating layer 7 is preferably composed of an insulating material. The reason resides in that since a distal portion of the coating layer 7 enters into the annular member 5 and the annular member 5 is located on the outer periphery of the coating layer 7, it is possible by forming the coating layer 7 from an insulating material to insulate the core wire 3 and the annular member 5 from each other. Consequently, for example where a medical implement to be used by passing an electric current is disposed along the guide wire 1, troubles such as leakage of current from the outer surface of the annular member 5 can be prevented from occurring.

Examples of the insulating material capable of reducing friction include polyolefins such as polyethylene, polypropylene, etc., polyvinyl chloride, polyesters (PET, PBT, etc.), polyamides, polyimides, polyurethane, polystyrene, polycarbonate, silicone resins, fluororesins (PTFE, ETFE, PFA, etc.), and composite materials thereof.

Of the these insulating materials, particularly, the fluororesins (and composite materials containing the same) can be used to reduce more effectively the frictional resistance (sliding resistance), and enhance the slidability, between the guide wire 1 and the inner wall of a catheter, thereby promising better steerability of the guide wire 1 in the catheter. In addition, this ensures that when the guide wire 1 is moved and/or rotated inside an endoscope, the guide wire 1 can be securely prevented from kinking or twisting.

The mean thickness of the coating layer 7 is not particularly limited, and is preferably, for example, 10 to 40 µm, more preferably 20 to 30 µm.

As shown in FIGS. 2 and 4, a primary layer 13 different in color from the marker 12 is formed between the marker 12 and the wire body 2. The material constituting the primary layer 13 is not particularly limited, and may for example be the same material as that of the coating layer 7. For instance, where a fluororesin (or a composite material containing the same) is used as the material for forming the primary layer 13, coating of the core wire 3 with the resin material can be conducted while heating the resin material, by a method such as baking and spraying. This promises excellent adhesion between the core wire 3 and the primary layer 13. Then, the marker 12 and the coating layer 7 are firmly attached to the core wire 3 through the primary layer 13.

The mean thickness of the primary layer 13 is not particularly limited, and is preferably, for example, 3 to 20 µm, more preferably 5 to 10 µm.

Incidentally, the outer surface of at least a distal portion of the guide wire 1 is preferably coated with a hydrophilic material. This ensures that the hydrophilic material produces lubricity through wetting, whereby the friction (sliding resistance) on the guide wire 1 is reduced, and the slidability is further enhanced. Consequently, the steerability of the guide wire 1 is further enhanced.

Examples of the hydrophilic material include cellulose polymer materials, polyethylene oxide polymer materials, maleic anhydride materials (for example, maleic anhydride copolymer such as methyl vinyl ether-maleic anhydride copolymer), acrylamide polymer materials (for example, polyacrylamide, polyglycidyl methacrylate-dimethylacrylamide (PGMA-DMAA) block copolymer), watersoluble nylon, polyvinyl alcohol, polyvinyl pyrrolidone, etc.

Such a hydrophilic material, in many cases, exhibits lubricity through wetting (absorbing water), to reduce the frictional resistance (sliding resistance) between the guide wire 1 and the inner wall of a catheter (tubular body) or an endoscope which is used together with the guide wire 1. As a result, slidability of the guide wire 1 is further enhanced, leading to better slidability of the guide wire 1 in the catheter.

### <Second Embodiment>

FIG. 7 is a side view showing a second embodiment of the guide wire according to the present invention.

Now, the second embodiment of the guide wire according to the present invention will be described below referring to the figure. The following description will be centered on the difference from the above-described embodiment, and description of the same items as above will be omitted.

This embodiment is the same as the first embodiment, excepting the conditions in which the first line-form portion and the second line-form portion are formed.

In a marker 12A of a guide wire 1A shown in FIG. 7, the pitch of helix of a first line-form portion 121 and the pitch of helix of a second line-form portion 122 are different from each other. In the configuration shown in the figure, the pitch of helix of the second line-form portion 122 is set to be smaller than the pitch of helix of the first line-form portion 121.

Such a configuration is effective in the case where the number of intersecting part 123 formed is to be set comparatively large.

In addition, the width (mean width) of the first line-form portion 121 and the width (mean width) of the second line-form portion 122 are different from each other. In the configuration shown, the width of the first line-form portion 121 is set to be larger (wider) than the width of the second line-form portion.

With such a configuration, the size of the intersecting parts 123 in this embodiment can be set to be greater than the size of the intersecting potions in the first embodiment. Consequently, the visibility of the intersecting parts 123 is enhanced, that is, the intersecting parts 123 can be made easier to look at.

### <Third Embodiment>

FIG. 8 is a side view of a third embodiment of the guide wire according to the present invention.

Now, the third embodiment of the guide wire according to the present invention will be described below referring to the figure. The following description will be centered on the difference from the above-described embodiment, and description of the same items as above will be omitted.

This embodiment is the same as the first embodiment, excepting the conditions in which the first line-form portion and the second line-form portion are formed.

In a marker 12B of a guide wire 1B shown in FIG. 8, the widths of a first line-form portion 121 and a second line-form portion 122 are gradually decreased along the distal direction. The portion where the widths are gradually decreased (gradually decreasing width portion) is preferably formed at a tapered portion 34 of a wire body 2. By checking such a portion, it is possible to grasp that the wire body 2 is decreasing in diameter, and is hence easily deformable (is high in flexibility, at the portion.

In addition, intersecting parts 123 differ in size in side view, according to the respective widths of the first line-form portion 121 and the second line-form portion 122. Such a configuration is effective in the case where the intersecting parts 123 are to be varied in size according to the outer diameter of the wire body 2. For example, as shown in FIG. 8, a configuration can be adopted in which the intersecting parts 123 are small in size at the tapered portion 34 of the wire body 2, whereas the intersecting parts 123 are large in size at the body portion 35 which is greater in outer diameter (mean outer diameter) than the tapered portion 34. This renders the intersecting parts 123 easy to look at.

While the guide wire according to the present invention has been described above referring to the embodiments shown in the drawings, the invention is not limited to the embodiments, and the component portions of the guide wire can be replaced by those of arbitrary configurations which can exhibit the same or equivalent functions. Besides, arbitrary components may be added to the above-described embodiments.

In addition, the guide wire according to the present invention may be a combination of two or more of the configurations (features) of the above-described embodiments.

Besides, the first line-form portion and the second line-form portion are not limited to those formed by drying a liquid material, and may for example be those formed by helically winding a belt-like (ribbon-like) member.

### [Industrial Applicability]

The guide wire according to the present invention includes an elongated wire body, and a marker which is provided at at least a distal-side portion of the wire body over the whole circumference thereof and which functions to indicate the intracorporeal position of the wire body. The marker has a first line-form portion and a second line-form portion which intersect each other at a plurality of locations to have a grid-like shape as a whole. For this reason, there are formed a plurality of intersecting parts where the first line-form portion and the second line-form portion intersect each other. Paying attention to (observing) a predetermined one of the intersecting parts, the predetermined intersecting part appears to move in the radial direction of the guide wire when the guide wire is rotated about its axis. Since the marker can be visually checked, when the guide wire is rotated about its axis the fact that "the guide wire is rotated" can be securely confirmed (grasped). In addition, when the guide wire is moved in its axial direction, the predetermined intersecting part appears to be moved along the axial direction of the guide wire, unlike in the case where the guide wire is rotated about its axis. Thus, when the guide wire is moved along its axis or rotated about its axis, the marker appears in different manners. Therefore, it is possible to securely distinguish whether the practical displacement of the guide wire is movement or rotation. Accordingly, when the guide wire is rotated about its axis, the operator can be securely prevented from having an illusion that the motion is a movement in the axial direction. Thus, the guide wire is excellent in steerability. In addition, where the marker is so formed that the intersecting parts of the first line-form portion and the second line-form portion are greater in height than the other parts, the outer surface of the guide wire is raised at the intersecting parts. This ensures that the area of contact between the outer surface of the guide wire and, for example, the lumen of a catheter or the lumen of an endoscope is reduced, whereby frictional resistance (sliding resistance) is reduced, slidability of the guide wire is enhanced, and steerability of the guide wire is improved. Therefore, the guide wire according to the present invention possesses industrial applicability.

## Claims

1. A guide wire (1) comprising:
an elongated wire body (2); and
a marker (12) which is provided at at least a distal-side portion of said wire body (2) over the whole circumference and which has a function of indicating the intracorporeal position of said wire body (2),
wherein said marker (12) has a first line-form portion (121) and a second line-form portion (122) which intersect each other at a plurality of locations to have a grid-like shape as a whole,
**characterized in that** the first line-form portion (121) and the second line-form portion (122) are each upheaved from a primary layer (13) and each have a semi-elliptic shape and a convexly curved top portion in vertical sectional view.

2. The guide wire as set forth in claim 1, wherein said first and second line-form portions have decreasing pitches of helix in the distal direction.

3. The guide wire as set forth in claim 1 or 2,
wherein said first and second line-form portions have different colors.

4. The guide wire as set forth in any of claims 1 to 3, wherein the widths of the first and second line-form portions gradually decrease in the distal direction.

5. The guide wire as set forth in any of claims 1 to 4, wherein said first line-form portion (121) has a helical shape, and said second line-form portion (122) has a helical shape with a winding direction of helix opposite to the winding direction of helix of said first line-form portion.

## Patentansprüche

1. Führungsdraht (1), umfassend:
einen langgestreckten Drahtkörper (2); und
eine Markierung (12), die an mindestens einem distalen Seitenabschnitt des Drahtkörpers (2) über den gesamten Umfang vorgesehen ist und die eine Funktion zum Anzeigen der intrakorporalen Position des Drahtkörpers (2) aufweist,
wobei die Markierung (12) einen ersten linienbildenden Abschnitt (121) und einen zweiten linienbildenden Abschnitt (122) aufweist, die sich an einer Vielzahl von Stellen schneiden, um eine gitterartige Form als Ganzes aufzuweisen, **dadurch gekennzeichnet, dass** der erste linienbildende Abschnitt (121) und der zweite linienbildende Abschnitt (122) jeweils aus einer Primärschicht (13) emporgehoben sind und jeweils eine halbelliptische Form und einen konvex gekrümmten oberen Abschnitt in der vertikalen Schnittansicht aufweisen.

2. Führungsdraht nach Anspruch 1, wobei der erste und zweite linienbildende Abschnitt abnehmende Steigungen der Spirale in der distalen Richtung aufweisen.

3. Führungsdraht nach Anspruch 1 oder 2,
wobei die ersten und zweiten linienbildenden Abschnitte unterschiedliche Farben aufweisen.

4. Führungsdraht nach einem der Ansprüche 1 bis 3, wobei die Breiten der ersten und zweiten linienbildenden Abschnitte allmählich in distaler Richtung abnehmen.

5. Führungsdraht nach einem der Ansprüche 1 bis 4, wobei der erste linienbildende Abschnitt (121) eine spiralförmige Form aufweist und der zweite linienbildende Abschnitt (122) eine spiralförmige Form mit einer Wickelrichtung der Spirale entgegengesetzt zur Wickelrichtung der Spirale des ersten linienbildenden Abschnitts aufweist.

## Revendications

1. Fil de guidage (1), comprenant :
un corps de fil (2) allongé et
un repère (12), qui est prévu sur au moins une partie côté distal dudit corps de fil (2), sur toute la circonférence et qui a une fonction, consistant à indiquer la position intracorporelle dudit corps de fil (2),
dans lequel ledit repère (12) a une première partie en forme de ligne (121) et une seconde partie en forme de ligne (122), qui se croisent mutuellement à une pluralité d'endroits, pour avoir une forme de grille dans son ensemble,
**caractérisé en ce que**
la première partie en forme de ligne (121) et la seconde partie en forme de ligne (122) sont soulevées chacune depuis une couche primaire (13) et ont chacune une forme semi-elliptique et une partie supérieure courbée de manière convexe dans une vue en coupe verticale.

2. Fil de guidage selon la revendication 1, dans lequel les dites première et seconde parties en forme de ligne ont des pas d'hélice décroissants dans la direction distale.

3. Fil de guidage selon la revendication 1 ou 2, dans lequel lesdites première et seconde parties en forme de ligne ont des couleurs différentes.

4. Fil de guidage selon l'une quelconque des revendications 1 à 3, dans lequel les largeurs des première et seconde parties en forme de ligne diminuent progressivement dans la direction distale.

5. Fil de guidage selon l'une quelconque des revendications 1 à 4, dans lequel ladite première partie en forme de ligne (121) a une forme hélicoïdale et ladite seconde partie en forme de ligne (122) a une forme hélicoïdale avec une direction d'enroulement d'hélice, opposée à la direction d'enroulement d'hélice de ladite première partie en forme de ligne.
